# EUROPEAN PATENT APPLICATION

(11) **EP 1 852 509 A1**
(43) Date of publication of application: **07.11.2007**
(21) Application number: 06713914.7
(22) Date of filing: 16.02.2006
(51) Int. Cl.: C12N 15/31, C07K 7/06, C07K 14/195, C12N 1/15, C12N 1/19, C12N 1/21, C12N 5/10, C12N 15/09, C12P 21/02, C12Q 1/32

(54) **NOVEL PROTEIN PROMOTING CARBONIC ACID FIXATION AND METHOD OF FIXING CARBONIC ACID BY USING THE PROTEIN**

(30) Priority: 18.02.2005 JP 2005042671
(71) Applicant: Asahi Glass Company, Limited, Chiyoda-ku, Tokyo 100-8405 (JP)
(72) Inventor: AOSHIMA, Miho, Tokyo 114003 (JP); TOHDA, Hideki, , Kanagawa-ku, Yokohama-shi, Kanagawa 22 (JP); HAMA, Yuko, , Kanagawa-ku, Yokohama-shi, Kanagawa 22 (JP)
(74) Representative: Müller-Boré & Partner Patentanwälte
(86) International application number: PCT/JP2006/302773
(87) International publication number: WO 2006/088110

(57) **Abstract**

The present invention provides a novel protein which allows efficient reductive carboxylation by isocitrate dehydrogenase, a method for efficiently producing the protein, and a method for efficient carboxylation using the protein.

A novel protein from Hydrogenobacter thermophilus TK-6 was found to promote the reductive carboxylation by isocitrate dehydrogenase, and the structure of the gene encoding the protein and the amino acid sequence of the protein were determined. It was found that it is possible to efficiently produce the protein by expressing the resulting gene abundantly. Further, it was found that the protein allows the reductive carboxylation by isocitrate dehydrogenase to proceed efficiently.

## Description

### TECHNICAL FIELD

The present invention relates to a novel protein which promotes the reductive carboxylation by isocitrate dehydrogenase, a method for producing the protein, or a method for carboxylation using the protein. The present invention also relates to a peptide or polypeptide entirely or partly containing the amino acid sequence of the protein, a polynucleotide encoding the peptide or polypeptide, a recombinant vector containing the polynucleotide, a transformant transformed with the recombinant vector and a method for producing a peptide or polypeptide using the transformant.

### BACKGROUND ART

Isocitrate dehydrogenase is an enzyme which catalyzes the oxidative decarboxylation of isocitrate to 2-oxoglutarate and widely distributed in the living world. Because carbon dioxide, one of the products of the reaction catalyzed by the enzyme, is released from the reaction system immediately after the reaction, the reverse reaction (the reductive carboxylation) hardly proceeds. The reductive carboxylation of 2-oxoglutarate to isocitrate is very disadvantageous in terms of energy. There are some reports that the enzyme catalyzes the reverse reaction (reductive carboxylation), though with very poor efficiency (non-patent documents 1-3).

Thus, carbon dioxide fixation utilizing the reversible catalysis by the enzyme is difficult and inefficient.
Non-patent document 1: Hathaway, J.A., an Atkinson, D.E. (1963). The effect of adenylic acid on yeast nicotinamide adenine dinucleotide isocitrate dehydrogenase, a possible metabolic control mechanism. J. Biol. Chem. 238, 2875-2881.
Non-patent document 2: B.D., Zink, M. W., and Stachow, C. S. (1964) Nicotinamide adenine dinucleotide-specific isocitric dehydrogenase. J. Biol. Chem. 239, 1597-1603.
Non-patent document 3: Kanao, T., Kawamura, M., Fukui, T., Atomi, H., and Imanaka, T (2002) Characterization of isocitrate dehydrogenase from the green sulfur bacterium Chlorobium Limicola. Eur. J. Biochem. 269, 1926-1931.

### DISCLOSURE OF THE INVENTION

### PROBLEMS THAT THE INVENTION IS TO SOLVE

There are no reports so far about proteins which promote the reductive carboxylation by isocitrate dehydrogenase. The object of the present invention is to provide a novel protein which allows efficient reductive carboxylation by isocitrate dehydrogenase, a method for efficiently producing the protein, and a method for efficient carboxylation using the protein. Another object of the present invention is to provide a peptide or polypeptide entirely or partly containing the amino acid sequence of the protein, a polynucleotide encoding the peptide or polypeptide, a recombinant vector containing the polynucleotide, a transformant transformed with the recombinant vector and a method for producing a peptide or polypeptide using the transformant.

### MEANS OF SOLVING THE PROBLEMS

To solve the above-mentioned problems, in the present invention, a novel protein from Hydrogenobacter thermophilus TK-6 which promotes the reductive carboxylation by isocitrate dehydrogenase was found, and the structure of the gene encoding the protein was determined. Then, it was found that it is possible to efficiently produce the protein by expressing the resulting gene abundantly. Further, the it was found that the protein allows the reductive carboxylation by isocitrate dehydrogenase to proceed efficiently. The present invention was accomplished on the basis of these discoveries.

Namely, the present invention provides:
1. A carboxylation promoting protein which promotes reductive carboxylation of 2-oxoglutarate to isocitrate by isocitrate dehydrogenase and contains two subunits having a molecular weight of about 72 kDa and a molecular weight of about 49 kDa.
2. An about 72-kDa carboxylation promoting protein subunit having any of the following sequences (1) to (4):
   (1) a polypeptide having an amino acid sequence shown as SEQ ID NO:1;
   (2) a polypeptide containing the polypeptide as identified above in (1);
   (3) a polypeptide having an amino acid sequence shown as SEQ ID NO:1 in which at least one amino acid is substituted, deleted, inserted and/or added.
   (4) a polypeptide having an amino acid sequence which is at least 70% homologous to SEQ ID NO:1.
3. A DNA having any one of the following sequences (1) to (3):
   (1) a polynucleotide encoding the polypeptide as identified above in 2 or its complementary strand:
   (2) a nucleic acid sequence shown as or complementary to SEQ ID NO:2: and
   (3) a DNA hybridizable to the DNA as identified above in (1) or (2) under stringent conditions.
4. An about 49-kDa carboxylation promoting protein subunit having a carbon dioxide fixing ability, which has any one of the following sequences (1) to (4):
   (1) a polypeptide having an amino acid sequence shown as SEQ ID NO:3;
   (2) a polypeptide containing the polypeptide as identified in (1);
   (3) a polypeptide having an amino acid sequence shown as SEQ ID NO:3 in which at least one amino acid is substituted, deleted, inserted and/or added; and
   (4) a polypeptide having an amino acid sequence at least 70% homologous to SEQ ID NO:3.
5. A DNA having a sequence selected from the following (1) to (3):
   (1) a polynucleotide encoding the polypeptide as identified above in 4 or its complementary strand;
   (2) a DNA having a nucleic acid sequence shown as SEQ ID NO:4 or its complementary strand; and
   (3) a DNA hybridizable to the DNA as identified above in (1) or (2) under stringent conditions.
6. A vector carrying the DNA as identified above in 3, the DNA as identified above in 5, or both the DNA as identified above in 3 and the DNA as identified above in 5.
7. A transformant carrying the DNA as identified above in 3, the DNA as identified above in 5, both the DNA as identified above in 3 and the DNA as identified above in 5, or the vector as identified above in 6.
8. A method for producing a carboxylation promoting protein, which comprises culturing the transformant as identified above in 7 and recovering an expressed protein.
9. A carboxylation promoting protein which comprises the carboxylation promoting protein subunit as identified above in 2 and the carboxylation promoting protein subunit as identified above in 4.
10. A carbon dioxide fixing method which comprises promoting reductive carboxylation by isocitrate dehydrogenase using the carboxylation promoting protein as identified above in 1 or 9.

### EFFECTS OF THE INVENTION

The present invention provides a novel protein which promote reductive carboxylation by isocitrate dehydrogenase, and the protein allows the enzymatic reaction by isocitrate dehydrogenase to proceed toward carbon dioxide fixation.

### BRIEF EXPLANATION OF THE DRAWINGS

[Fig. 1] A restriction map around the gene (cfiAB) of a carbon dioxide fixing protein from Hydrogenobacter thermophilus TK-6. There is another open reading (orf1) upstream (Example 2).
[Fig. 2] 10% SDS-PAGE of 1.5 µg (lane 1) of a recombinant carbon dioxide fixing protein from Escherichia coli followed by Coomassie staining. Lane M is a size marker. (Example 4)
[Fig. 3] The isocitrate concentrations in 200 µl reaction solutions containing 2.3 µg of the purified recombinant carbon dioxide fixing protein after 2, 4, 6, 10 and 20 minutes of reaction. (Example 5)
[Fig. 4] (a) A chromatogram of a 3-fold dilution of a low-molecular-weight fraction of 200 µl of a reaction solution containing 2.3 µg of the purified carbon dioxide fixing protein after 6 minutes of reaction. Arrow 1 indicates the peak of bicarbonate ions, arrow 2 indicates 2-oxglutarate, arrow 3 indicates phosphate, arrow 4 indicates NADH, arrow 5 indicates isocitrate, arrow 6 indicates ADP, and arrow 7 indicates ATP. (b) A chromatogram of a standard solution containing 0.1 mM isocitrate and 0.1 mM ADP. (Example 5)
[Fig. 5] 10%. SDS-PAGE of a size marker (lane M), 1.5 µg of the recombinant carbon dioxide fixing protein (lane 1) and 0.3 µg of the recombinant carbon dioxide fixing protein (lane 2). For the lanes M and 1, the electrophoresis was followed by Coomassie staining. The lane 2 was blotted onto a PVDF membrane, and the biotinylated polypeptide was stained with a streptavidin-HRP conjugate. The Coomassie-stained area (lanes M and 1) and the Western-blotted area (lane 2) are shown together. (Example 6)
[Fig. 6] Molecular weight analysis using a Superrose 6 column. Solid circles indicate the elution volumes of 5 proteins in Bio-Rad gel permeation standard. The arrow indicates the elution volume of the carbon dioxide fixing protein. (Example 7)
[Fig. 7] Isoelectric focusing of 4.5 µg of the purified recombinant carbon dioxide fixing protein from E. coli (lane 1) and 4.5 µg of the purified carbon dioxide fixing protein from Hydrogenobacter thermophilus TK-6 (lane 2). Lane M is an isoelectric point marker. (Example 8)
[Fig. 8] The pH dependence of the carboxylation reaction in the presence of the carboxylation promoting protein. The carboxylation activity was assayed at room temperature in reaction solutions prepared by using a 1 M Bicine-KOH buffer stock. The correct pH values were measured at 70°C after addition of all the substrates to the reaction solutions. (Example 9)
[Fig. 9] The temperature dependence of the carboxylation reaction in the presence of the carboxylation promoting protein. The carboxylation rate was measured at reaction temperatures of 40°C, 50°C, 60°C, 70°C, 80°C and 90°C. (Example 10)
[Fig. 10] The heat resistance of the carboxylation promoting protein. After 10 minutes of heat treatment of the carboxylation promoting protein at 50°C, 60°C, 70°C, 72°C, 76°C, 80°C, 90°C and 95°C, the residual activity was measured at 70°C. (Example 11)
[Fig. 11] An S-V curve at 2-oxoglurarate concentrations from 0.2 mM to 5 mM. (Example 12)
[Fig. 12] An S-V curve at Mg-ATP concentrations from 0.2 mM to 5 mM. (Example 12)
[Fig. 13] An S-V curve at a 10 mM Na-ATP concentration at magnesium sulfate concentrations from 2 mM to 100 mM. (Example 12)
[Fig. 14] An S-V curve at a 10 mM magnesium sulfate concentration at Na-ATP concentrations from 0.2 mM to 20 mM. (Example 12)

### BEST MODE FOR CARRYING OUT THE INVENTION

### (Novel Protein)

The novel protein of the present invention acts to promote the enzymatic reaction by isocitrate dehydrogenase toward carboxylation (hereinafter referred to also as a carboxylation promoting protein), i.e., promotes the reductive carboxylation of 2-oxoglutarate to isocitrate by isocitrate dehydrogenase. The carboxylation promoting protein can be isolated from Hydrogenobacter thermophilus TK-6, though it may be of any origin without any particular restrictions as long as it has the above-mentioned activity. According to structural analysis by SDS-PAGE, the carboxylation promoting protein consists of a subunit of about 72 kDa and a subunit of about 49 kDa in a ratio of 1:1.

### (Polypeptide)

The Hydrogenobacter thermophilus TK-6 carboxylation promoting protein of the present invention has amino acid sequences shown as SEQ ID NO:1 and SEQ ID NO:3, but may not have the above-mentioned amino acid sequence as long as it promotes the enzymatic reaction by isocitrate dehydrogenase toward carboxylation.

The carboxylation promoting protein of the present invention consists of a polypeptide having an amino acid sequence shown as SEQ ID NO: 1 in the Sequence Listings constituting the subunit of about 72 kDa of the carboxylation promoting protein and a polypeptide having an amino acid sequence shown as SEQ NO:3 constituting the subunit of about 49 kDa. Further, the polypeptide of the present invention is a polypeptide having at least part of SEQ ID NO:1 or SEQ ID NO:3. The polypeptide has an amino acid sequence at least about 40%, preferably at least about 70%, more preferably at least about 90%, particularly preferably at least about 95%, similar to SEQ ID NO:1 or SEQ ID NO:3. Selection of a polypeptide having such similarity may be based on whether it functions as a subunit of the carboxylation promoting protein, by following the procedure in Example 5.

The amino acid sequence similarity may be determined by known techniques, for example, by direct amino acid sequencing, or by cDNA sequencing followed by deduction of the encoded amino acid sequence.

The polypeptide of the present invention covers polypeptides having at least part of the amino acid sequence shown as SEQ ID NO:1 or SEQ ID NO:3 which can be used as reagents, standards or immunogens. The present invention covers polypeptides having amino acid sequences consisting of at least 8, preferably at least 10, more preferably at least 12, and particularly preferably at least 15 serially joined amino acids, which preferably are immunologically immobilizable. These peptides may be used as reagents or standards, or may be used as antigens for preparation of antibodies against the carboxylation promoting protein of the present invention singly or after combined with carriers (such as keyhole limpet hemocyanin or ovalbumin). The present invention covers those linked to other proteins or substances.

Further, on the basis of the specific polypeptides, it is possible to provide polypeptides having amino acid sequences containing mutations by deleting, substituting, adding or inserting from 1 to 100, preferably from 1 to 30, more preferably from 1 to 20, particularly preferably 1 or several amino acids, as long as they have the ability to promote the enzymatic reaction by isocitrate dehydrogenase toward carboxylation. Deletions, substitutions, additions and insertions can be introduced by known techniques such as site-specific mutagenesis, homologous recombination, primer extension or polymerase chain reaction (PCR) or combinations thereof as disclosed in "Molecular Cloning, A Laboratory Manual 2nd ed." edited by Sambrook et al. (Cold Spring Harbor Laboratory Press) 1989, "Labo Manual Genetic Engineering" edited by Masami Muramatsu (Maruzen) 1988, "PCR Technology: Principles and applications for DNA amplification" edited by Erlich HE (Stockston Press) 1989 or other books, optionally with some modifications, and for example, Ulmer's techniques (Science, 219, 666, 1983) may be used.

When the above-mentioned mutations are introduced, it is easily supposed that substitutions with analogous amino acids (polar, non-polar, hydrophobic, hydrophilic, positively charged, negatively charged or aromatic amino acids) do not change the basic properties (such as physical properties, activity or immunological activity) of the protein.

The present invention also provides a polypeptide which promotes the enzymatic reaction by isocitrate dehydrogenase toward carboxylation like the protein consisting of polypeptides having amino acid sequences shown as SEQ ID NO:1 and SEQ ID NO:3, or its minimum active element (region or domain) as well as a polypeptide with modified activity. These are useful for screening of substances for ability to promote the enzymatic reaction by isocitrate dehydrogenase toward carboxylation.

Further, it is easy for a person skilled in the art to attach another protein such as alkaline phosphatase, β-galactosidase, the Fc fragments of Ig G or other immunoglobulins or FLAG-tag or other peptides directly or via a linker peptide to the N-terminus or C-terminus through genetic engineering in order to facilitate detection or isolation of the polypeptide of the present invention or impart another function, and the present invention also covers such polypeptides bonded to these different substances.

### (Polynucleotide)

In one embodiment, the polynucleotide of the present invention and its complementary strand mean a polynucleotide encoding the amino acid sequence of the polypeptide of the present invention, for example, SEQ ID NO:1 or SEQ ID NO:3 in the Sequence Listings and its complementary strand. They provide genetic information useful for production of the above-mentioned carboxylation promoting protein and can be used as nucleic acid reagents or standards. The sequence of the polynucleotide is preferably SEQ ID NO:2 or SEQ ID NO:4 or its complementary strand.

In another embodiment, the present invention provides a polynucleotide hybridizable to the nucleotide encoding the amino acid sequence of the polypeptide of the present invention, for example, the amino acid sequence shown as SEQ ID NO:1 or SEQ ID NO:3 in the Sequence Listings, preferably a polynucleotide having a nucleic acid sequence shown as SEQ ID NO:2 or SEQ ID NO:4 or the corresponding region of its complementary strand under stringent conditions. The hybridization conditions disclosed, for example, in "Molecular Cloning, A Laboratory Manual 2nd ed." edited by Sambrook et al. (Cold Spring Harbor Laboratory Press) 1989, may be emploted. These polynucleotides may not necessarily be complementary to the target polynucleotide, especially the polynucleotide having a nucleic acid sequence shown as SEQ ID NO:2 or SEQ ID NO:4 or its complementary strand, as long as they hybridize to them and, for example, have at least about 40%, for example at least about 70%, preferably at least about 80%, more preferably at least about 90%, particularly preferably at least 95% sequence similarity to the polynucleotide having a nucleic acid sequence shown as SEQ ID NO:2 or SEQ ID NO:4 or its complementary strand. Further, the polynucleotide of the present invention covers polynucleotides and oligonucleotides consisting of at least 10, preferably at least 15, more preferably 20, particularly preferably at least 30 consecutive nucleotides within the above-mentioned nucleic acid sequences and their complementary strands.

These polynucleotides are useful as probes or primers for detection of the nucleic acid encoding the carboxylation promoting protein such as its gene or mRNA or as antisense oligonucleotides for regulation of gene expression in production of the polypeptide of the present invention. Here, the nucleotide sequence encoding the carboxylation promoting protein or a polypeptide having similar activity can be determined, for example, by screening proteins expressed by a known protein expression system for the ability to promote the enzymatic reaction by isocitrate dehydrogenase toward carboxylation.

The carboxylation promoting protein may be assembled from two subunits separately expressed from the polynucleotides encoding them, but is preferably assembled by coexpressing the two subunits from a polynucleotide obtained by joining the nucleic acid sequences encoding them.

### (Transformant)

The present invention can provide the carboxylation promoting protein of the present invention or the polypeptides constituting it, by using recombinant DNA techniques using known hosts such as prokaryotic cells including E. coli and Bacillus subtilis, eukaryotic cells including budding yeasts, fission yeasts and filamentous fungi, insect, animal or plant cells, insects, animals and plants without any restrictions, although E. coli was used in the Examples of the present invention. For transformation, known techniques may be used, and for example, the host is transformed by using a plasmid, a chromosome, a virus or the like as the replicon. Chromosomal integration is preferred in view of gene stability, but autonomous replication using an extranuclear gene is used for simplicity. A vector is selected for the selected host and comprises the sequence of the gene to be expressed and sequences carrying genetic information relating to the replication and regulation of the gene. A promoter, a ribosome binding site, a terminator, a signal sequence, an enhancer and the like may be used in an appropriate combination for a prokaryote or a eukaryote by a known method.

The transformant is incubated under culture conditions known to be appropriate for the host. The incubation may be carried out by following the ability to promote the enzymatic reaction by isocitrate dehydrogenase of the expressed carboxylation promoting protein or a polypeptide constituting it toward carboxylation, or the transformant may be subcultured or produced batchwise by following the transformant mass in the culture.

### (Purification)

The carboxylation promoting protein of the present invention may be purified directly from cells producing the protein, or may be obtained as a recombinant by expressing the gene encoding the protein in a heterologous host. For example, it is possible to express a gene encoding the amino acid sequences of Hydrogenobacter thermophilus Tk-6 carboxylation promoting protein shown as SEQ ID NO:1 and SEQ ID NO:3 or encoding the amino acid sequences shown as SEQ ID NO:1 and SEQ ID NO:3 wherein at least one amino acid is deleted, substituted or added, and then recover the carboxylation promoting protein from the recombinant.

The carboxylation promoting protein of the present invention can be isolated from a culture of cells producing it or from a culture of host cells expressing a gene encoding the protein. Hydrogenobacter thermophilus TK-6 carboxylation promoting protein can be purified by subjecting a cell extract from the organism to hydrophobic chromatography, affinity chromatography, anion exchange chromatography and gel filtration column chromatography. When the protein is expressed in an E. coli host, the protein can be purified by subjecting a cell extract to heat treatment, hydrophobic chromatography and anion exchange chromatography. However, the method of purifying the protein is not restricted thereto, and it may be purified by any methods such as ammonium sulfate precipitation, ethanol precipitation, cation exchange chromatography and hydroxyapatite chromatography.

The carbon dioxide fixing method of the present invention is carried out by incubating a reaction solution containing 2-oxoglurarate, sodium bicarbonate, NADH, Mg-ATP and isocitrate dehydrogenase after addition of the carboxylation promoting protein obtained by the above-mentioned method so as to promote carbonation.

Screening for the novel carboxylation promoting protein of the present invention can be carried out by using the reductive carboxylation from 2-oxoglutarate to isocitrate by isocitrate dehydrogenase as the marker.

Specifically, it comprises the following steps:
(1) incubating reaction solutions containing candidate proteins, 2-oxoglutarate, sodium bicarbonate, NADH, MG-ATP and isocitrate dehydrogenase; and
(2) measuring reductive carboxylation from 2-oxoglutarate to isocitrate by isocitrate dehydrogenase.

### EXAMPLES

Now, the present invention will be described in further by referring by reference to the following Examples. However, the present invention is by no means restricted to these specific Examples.

### (EXAMPLE 1: Purification of a Carboxylation promoting protein)

8 g of wet cells of Hydrogenobacter thermophilus TK-6 were suspended in 30 mL of 20 mM Tris-HCl (pH 8.0) and disrupted by sonication. The cell debris was removed by centrifugation (15,000 g, 20 minutes), and the supernatant was desalted by dialysis against 20 mM Tris-HC1 (pH 8.0) and subjected to a DE52 column (Whatman). After washed with 20 mM Tris-HCl (pH 8.0), the column was eluted with 20 mM Tris-HCl (pH 8.0) containing 1 M NaCl. The eluate was dialyzed against 20 mM Tris-HCl (pH 8.0), and after 30% saturation with ammonium sulfate and loaded onto a Butyl-Toyoperal column (Tosoh). After washed well with 20 mM Tris-HCl containing 30% saturation of ammonium sulfate, the column was eluted with 20 mM Tris-HCl (pH 8.0) with an ammonium sulfate gradient from 30% to 0% saturation. The active fractions were dialyzed against 20 mM Tris-HCl (pH 8.0), passed through an AF Blue Toyopearl column (Tosoh) and then loaded onto a MonoQ column (Pharmacia). The column was washed well with 20 mM Tris-HCl (pH 8.0) and eluted with a NaCl gradient from 0M to 0.5 M. The active fractions were loaded onto a Superose6 column (Pharmacia) equilibrated with 20 mM Tris-HCl (pH 8.0) containing 200 mM NaCl. The resulting active fractions were dialyzed against 10 mM potassium phosphate buffer (pH 7.5) to give 3 mg of a purified protein. The purified protein gave two bands, about 72 kDa and about 49 kDa, in a 1:1 ratio in SDS-PAGE. This suggests that the protein consists of two kinds of subunits. The subunits separated by SDS-PAGE were blotted onto a PVDF membrane (Bio-Rad, Sequi-Blot) and sequenced with an amino acid sequencer (Applied Biosystems model 491). As a result, a sequence of MQAVEIMEEIREKFKEFEKGGFRKKILITD (SEQ ID NO:5 in the Sequence Listings) was obtained from the larger subunit, and a sequence of MFKKVLVANRGEIA(C)RVIRA(C)KELGIQTVA (SEQ ID NO:6 in the Sequence Listings) was obtained from the smaller subunit.

### (EXAMPLE 2: Isolation and Sequencing of the Gene Encoding the Carboxylation promoting protein)

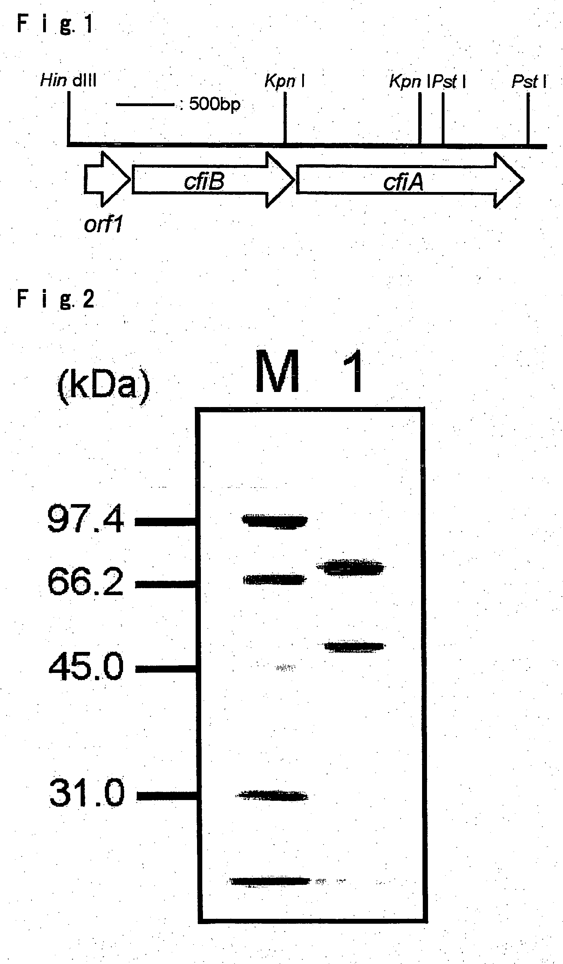

On the basis of the N-terminal amino acid sequence of the smaller subunit of the carboxylation promoting protein, a forward primer having a sequence of 5'-ATGTTYAARAARGTNYTNGTNGCNA-3' (SEQ ID NO:7 in the Sequence Listings) was synthesized, and a reverse primer having a sequence of 5'-CYTTYTCRAAYTCYTTRAAYTTYTC-3' (SEQ ID NO:8 in the Sequence Listings) was synthesized. PCR was carried out using the two synthetic primers and the genomic DNA of Hydrogenobacter thermophilus TK-6 as the template. The resulting PCR fragment was sequenced, and as a result, it turned out to contain the gene of the smaller subunit of the carboxylation promoting protein. Screening of a genomic library of Hydrogenobacter thermophilus TK-6 using the PCR fragment as a probe gave a fosmid clone hybridizable to the probe. A 4184-bp region containing the genes of both subunits of the carboxylation promoting protein within the insert sequence (about 30 kbp) in the fosmid clone were sequenced bidirectionally. The restriction map of the sequenced region is shown in Fig. 1. The sequences of the genes of the two subunits of the carboxylation promoting protein are shown as SEQ ID NO:2 and SEQ ID NO:4, and the amino acid sequences translated from them are shown as SEQ ID NO:1 and SEQ ID NO:3.

### (EXAMPLE 3: Construction of an Expression Plasmid)

An expression vector for expression of Hydrogenobacter thermophilus Tk-6 carboxylation promoting protein in E. coli as the host was constructed using T7-promoter. PCR using a fosmide clone containing the genes of both subunits of the carboxylation promoting protein as the template, a forward primer having a sequence of 5'-TTTGGAGGCACATATGTTTAAGAAGG-3' (SEQ ID NO:9 in the Sequence Listings) and a reverse primer having a sequence of 5'-ATGTACTACACCTCTAGAGTTTTTATCAA-3' (SEQ ID NO:10 in the Sequence Listings) was carried out after introduction of a NdeI site as the initiation codon and introduction of a XbaI site downstream of the termination codon to amplify the gene of the smaller subunit of the carboxylation promoting protein. PCR using a fosmide clone containing the genes of both subunits of the carboxylation promoting protein as the template, a forward primer having a sequence of 5'-TTGATAAAAACTCTAGAGGTGTAGTACAT-3' (SEQ ID NO:11 in the Sequence Listings) and a reverse primer having a sequence of 5'-GGTAAGGCTTCTCGAGATGGTTCAG-3' (SEQ ID NO:12 in the Sequence Listings) was carried out after introduction of a XbaI site upstream of the initiation codon and a XhoI site downstream of the termination codon to amplify the larger subunit of the carboxylation promoting protein. The PCR fragment was linked to a plasmid vector (pBluescript, Stratagene) and sequenced, and clones with no PCR errors (one plasmid clone containing the gene of the smaller subunit of the carboxylation promoting protein and one plasmid clone containing the gene of the larger subunit of the carboxylation promoting protein) were selected. From the plasmid clones, the gene of the smaller subunit of the carboxylation promoting protein and the gene of the larger subunit of the carboxylation promoting protein were obtained as a NdeI-XbaI fragment and as a XbaI-XhoI fragment, respectively, and inserted between the NdeI and XhoI sites of an expression vector pET21c (Novagen). The resulting expression vector was designated as pET21-CFI.

### (EXAMPLE 4 : Purification of a Recombinant Carboxylation promoting protein)

pET21-CFI was introduced into E. coli BL21 (DE3), and the resulting transformant was inoculated in 50 ml of 2xYT medium containing ampicillin and grown at 37°C overnight with shaking to give a preculture. 50 ml of the preculture was added to 1.5 liters of 2×YT medium containing ampicillin and grown at 37°C for 37hours with shaking, and after addition of IPTG to a final concentration of 0.5 mM, was grown at 37°C for another 3 hours with shaking. The resulting cells were suspended in 30 ml of 20 mM Tris-HCl (pH 8.0) contanining 1 mM magnesium chloride (hereinafter referred to as "magnecium chloride-containing buffer") and disrupted by sonication. ATP was added to the resulting suspension to a final concentration of 1 mM. After 20 minutes of heat treatment at 75°C, the cell debris was removed by centrifugation, and the supernatant was diluted with magnesium chloride-containing buffer by a factor of 6 and loaded onto a DE52 column (Whatman). The column was washed with magnesium chloride-containing buffer and eluted with magnesium chloride-containing buffer containing 1 M NaCl. After 30% saturation with ammonium sulfate, the eluted fraction were loaded onto a Butyl-Toyopearl column (Tosoh). The column was washed well with magnesium chloride-containing buffer and eluted with a NaCl gradient from 0 M to 0.5 M. The resulting active fraction was dialyzed against magnesium chloride-containing buffer and then loaded onto a MonoQ column (Pharmacia). The column was washed well with magnesium chloride-containing buffer and eluted with a NaCl gradient from 0 M to 5 M. The resulting active fraction was dialyzed against 10 mM Tris-HCl (pH 8.0) to give 1.7 mg of a purified protein. The purified recombinant carboxylation promoting protein gave two bands, about 72 kDa and about 49 kDa, in a 1:1 ratio in SDS-PAGE (Fig. 2).

### (EXAMPLE 5: Promotion of Carboxylation by Carboxylation promoting protein)

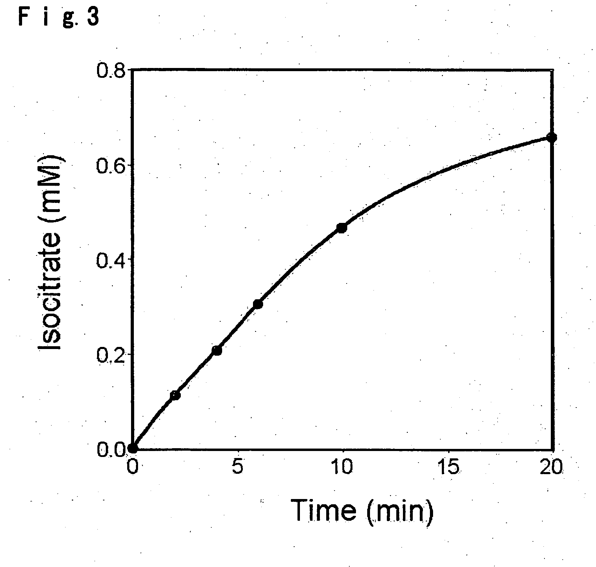

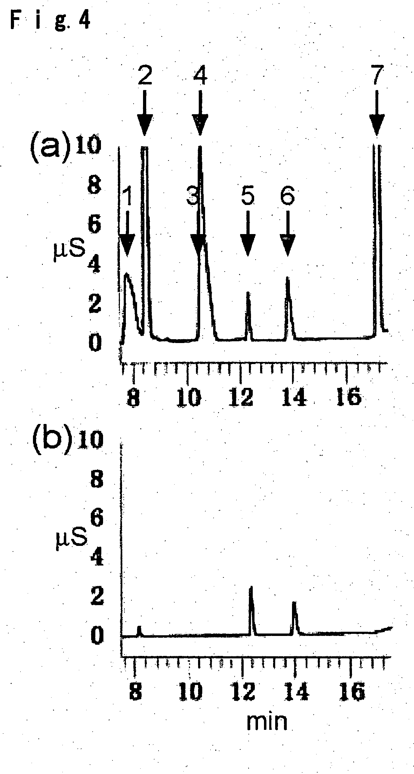

The activity of the carboxylation promoting protein was measured in 200 µl of a reaction solution containing 100 mM Bicine-KOH (pH 8.5), 50 mM sodium bicarbonate, 5 mM 2-oxoglurarate, 5 mM Mg-ATP, 4 mM NADH and 14 µg isocitrate dehydrogenase (a recombinant enzyme of Hydrogenobacter thermophilus TK-6 origin, isolated from E. coli) at 70°C. The reaction solution was preincubated in a glass microtube without Mg-ATP at 70°C for 2 minutes, and then Mg-ATP was added to initiate the reaction. The microtube was cooled with ice to stop the reaction at constant intervals, and the reaction solution was subjected to a cartridge with a cut-off molecular weight of 5000 (Ultrafree MC, Millipore). The resulting low-molecular-weight fraction was diluted appropriately with Milli Q water (Millipore) and subjected to ion chromatography (Dionex DX 500, column Ionpac AS11, Suppressor ASRS Ultra II, flow rate 2 ml/min). The column was equilibrated with 0.5 mM NaOH, eluted isocratically with 0.5 mM NaOH from 0 to 2 minutes, with a NaOH gradient from 0.5 mM to 5 mM from 2 to 5 minutes, with a NaOH gradient from 5 mM to 38.25 mM from 5 to 15 minutes and with a NaOH gradient from 38.25 mM to 100 mM from 15 to 16 minutes. The carboxylation product (isocitrate) was calculated from peak areas by comparison with a chromatogram of known concentrations of citrate. The results of determination of the isocitrate concentrations after 2, 4, 6, 10 and 20 minutes of reaction in the presence of 2.3 µg of the purified recombinant carboxylation promoting protein are shown in Fig. 3. Fig. 4 is the chromatogram of a threefold dilution of the low-molecular-weight fraction from the reaction solution after 6 minutes of reaction. The isocitrate formation was dependent on time and the amount of the carboxylation promoting protein. ATP hydrolysis was observed as the reaction proceeded. In the absence of the carboxylation promoting protein, isocitrate formation was not observed. The carboxylation promoting protein, isocitrate dehydrogenase, NADH and Mg-ATP were all essential for carboxylation. This suggests that the protein promotes reductive carboxylation by isocitrate dehydrogenase Mg-ATP-dependently.

### (EXAMPLE 6: Detection of Biotinylated Subunit)

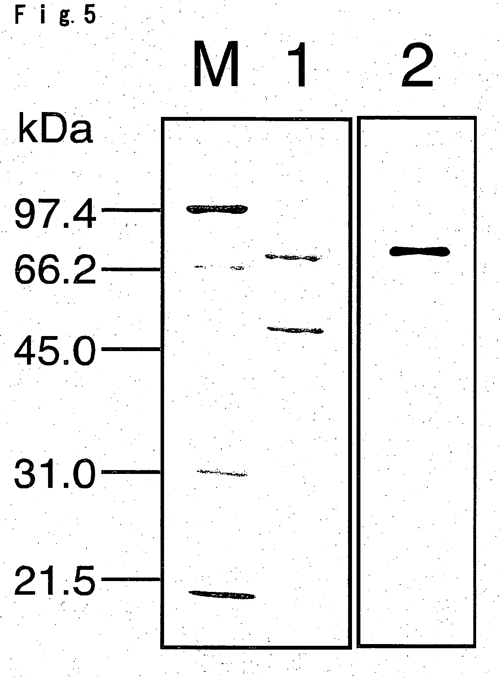

Carboxylation by the carboxylation promoting protein was completely inhibited by 1 U of avidin (Sigma), which suggests that the carboxylation promoting protein is a biotin protein. 0.3 µg of the purified recombinant carboxylation promoting protein was separated by 10% SDS-PAGE and blotted onto a PVDF membrane (Bio-Rad, Sequi-Blot) and reacted with a streptavidin-HRP conjugate (Pharmacia) to detect biotinylated polypeptides. As a result, a single band was detected (Fig. 5, lane 2). By comparison with the mobilities of a molecular weight marker (lane M) and 1.5 µg of the purified recombinant carboxylation promoting protein (lane 1) in electrophoresis on the same gel following by CBB staining, it turned out that the band corresponds to the large subunit. Namely, it turned out that among the two subunits of the protein, only the larger subunit is biotinylated. Similar experimentation demonstrated that the larger subunit of the carboxylation promoting protein isolated from Hydrogenobacter thermophilus TK-6 was biotinylated.

### (EXAMPLE 7: Molecular Weight Analysis by Gel Filtration)

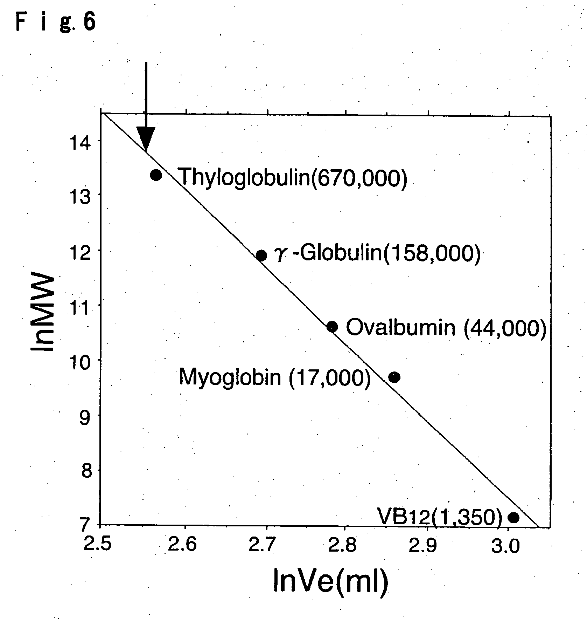

To estimate the molecular weight of the carboxylation promoting protein, gel filtration was performed using a Superrose column (Pharmacia) and 20 mM Tris-HCl (pH 8.0) containing 200 mM NaCl as the eluent. The column was equilibrated with the eluent, and 100 µl of a sample was injected. The eluting position of the protein (Ve) was determined by monitoring the absorbance at 280 nm. As a molecular weight marker, Gel Filtration Standard (Bio-Rad) was used. The carboxylation promoting protein eluted earlier than Thyloglobulin (670 kDa), which is the largest molecule in Gel Filtration Standard. The arrow in Fig. 6 indicates the eluting position of the carboxylation promoting protein. The carboxylation promoting protein isolated from TK-6 and the recombinant carboxylation promoting protein isolated from E. coli were eluted at the same position which corresponds to a molecular weight of from 900 to 1000 kDa. The estimated molecular weight was outside the range calibratable by Gel Filtration Standard and hence may not be accurate, but it suggests that the carboxylation promoting protein is a massive complex.

### (EXAMPLE 8: Isoelectric Point Determination)

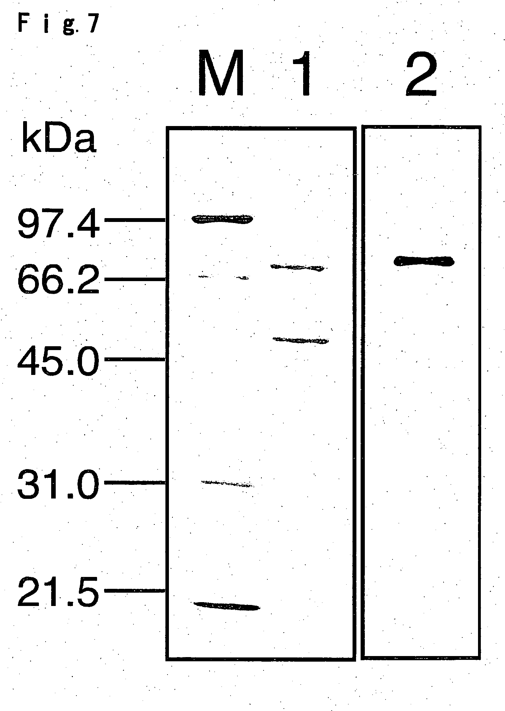

To estimate the isoelectric point of the carboxylation promoting protein, isoelectric focusing was carried out using a gel composed of 4.85% acrylamide, 0.15% N,N'-methylenebisacrylamide, 16.9% glycerin and 2.67% Ampholine (Pharmacia, pH 4.0-6.5 preblended), an Electrophoresis system Resolmax IEF (ATTO, AE-3230), 0.5 M phosphate solution containing 0.1 M glutamic acid as the anolyte, and 0.1 M β-alanine as the catholyte. Isoelectroric focusing was performed at 1000 V for 1 hour, at 1500 V for 1 hour and at 2000 V for 1.5 hours with cooling water circulating at 4°C. After the focusing, a linear pH gradient was confirmed by direct measurement of the pH of the gel. The isoelectric focusing was followed by fixation with 10% trichloroacetic acid containing 5% sulfosalicylic acid and CBB staining. The resulting electropherogram is shown in Fig. 7. The recombinant carboxylation promoting protein isolated from E. coli (lane 1) and the carboxylation promoting protein isolated from TK-6 (lane) showed the same mobility. From the actual pH of the gel and the mobility of an isoelectric point marker (Bio-Rad), the isoelectric point was estimated at 4.9.

### (EXAMPLE 9: Optimum pH)

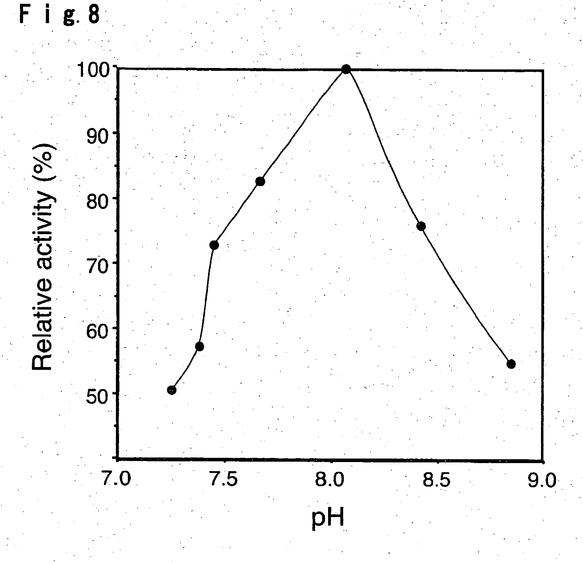

To estimate the optimum pH for the carboxylation promoting protein, carboxylation was carried out at various pH. 1 M Bicine-KOH buffer stocks adjusted at pH 6.5, 7.0, 7.5, 8.0, 9.0 and 9.5 at room temperature were prepared and added in a 1/10 volume to reaction solutions. The reaction was carried out in 200 µl of reaction solutions containing 100 mM Bicine-KOH buffer, 50 mM sodium bicarbonate, 5 mM 2-oxoglutarate, 5 mM Mg-ATP, 4 mM NADH, 14 µg of isocitrate dehydrogenase (a recombinant enzyme of Hydrogenobacter thermophilus TK-6 origin, isolated from E. coli) and 2.3 µg of a carboxylation promoting protein at 70°C. The reaction solutions were incubated in glass microtubes at 70°C for 4 minutes and then cooled with ice to stop the reaction. The carboxylation activity was assayed by determining the isocitrate formation was determined by ion chromatography. The actual pH's of the reaction solutions prepared from the stocks adjusted at pH 6.5, 7.0, 7.5, 8.0, 9.0 and 9.5 measured in the presence of all the substrates at 70°C were pH 7.25, pH 7.38, pH 7.45, pH 8.07, pH 8.42 and pH 8.85, respectively. The relative carboxylation activities (maximum activity 100%) measured in these reaction solutions were plotted, and the results are shown in Fig. 8. The optimum pH for the reaction is around 8, and the maximum activity was attained in the reaction solution prepared from a Bicine-KOH buffer stock at pH 8.5.

### (EXAMPLE 10: Optimum Temperature)

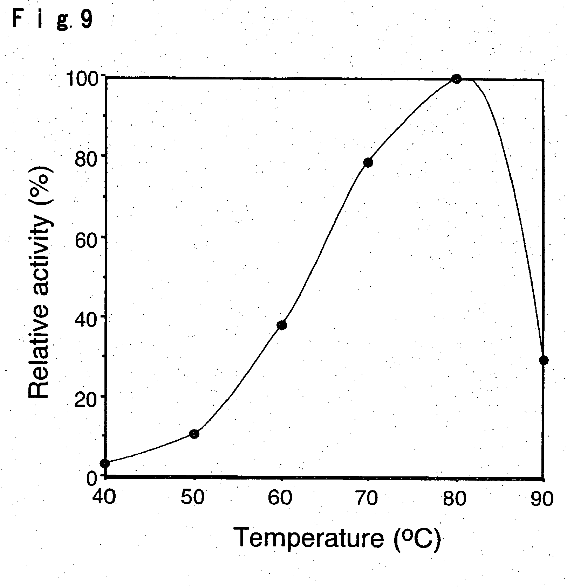

To estimate the optimum temperature for carboxylation promoted by the carboxylation promoting protein, carboxylation was carried out at various reaction temperatures. The reaction was carried out in 250 µl of reaction solutions containing 100 mM Bicine-KOH buffer, 50 mM sodium bicarbonate, 10 mM 2-oxoglutarate, 10 mM Mg-ATP, 18 µg of isocitrate dehydrogenase (a recombinant enzyme of Hydrogenobacter thermophilus TK-6 origin, isolated from E. coli), 0.2 mM NADH and the recombinant carboxylation promoting protein (2.3-5.7 µg). The reaction solutions were preincubated without NADH and the carboxylation promoting protein, and NADH and the carboxylation promoting protein were added to initiate the reaction. The decrease in absorbance at 340 nm (during initial 1 minute) was measured. The carboxylation rates at reaction temperatures of 40°C, 50°C, 60°C, 70°C, 80°C and 90°C are shown in Fig. 9. At 90°C, the reaction stopped within 30 seconds. The optimum temperature for carboxylation promoted by the carboxylation promoting protein was around 70 to 80°C.

### (EXAMPLE 11: Heat Resistance)

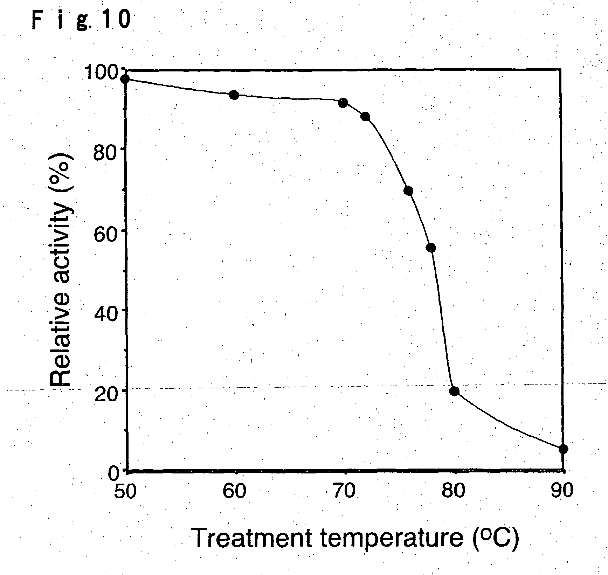

To estimate the heat resistance of the carboxylation promoting protein, carboxylation was carried out after 10 minutes of heat treatment at various temperatures. 50 µl of 50 mM HEPES-KOH buffer (pH 7.5) containing 250 µg/ml of the recombinant carboxylation promoting protein was dispensed into 1.5 mL tubes (Treff). The tubes were incubated at 50°C, 60°C, 70°C, 72°C, 76°C, 78°C, 80°C, 90°C and 95°C for 10 minutes and cooled rapidly with ice-cold water. The insolubles were removed by centrifugation; and carboxylation rate was determined using 10 µl of the supernatants (corresponding to 2.5 µg of the carboxylation promoting protein). Carboxylation was carried out in 250 µl of reaction solutions containing 100 mM Bicine-KOH buffer (pH 7.5), 50 mM sodium bicarbonate, 10 mM 2-oxoglutarate, 10 mM Mg-ATP and 18 µg of isocitrate dehydrogenase (a recombinant enzyme of Hydrogenobacter thermophilus TK-6 origin, isolated from E. coli) at 70°C. the reaction solutions were preincubated for 5 minutes, and 0.2 mM NADH and the heat treated carboxylation promoting protein were added to initiate the reaction. The decrease in absorbance at 340 nm (during initial 1 minute) was measured. The residual activity based on the activity before heat treatment was plotted against heat treatment temperature, and the results are shown in Fig. 10. The protein retained about 90% of its activity even after 10 minutes of heat treatment at 70°C. From the profile obtained, it is estimated that the protein loses 50% of its activity at about 78.3°C.

### (EXAMPLE 12: Measurement of Kinetic Parameters)

To determine kinetic parameters for the carboxylation promoting protein, carboxylation was carried out at various concentrations of 2-oxoglurarate and Mg-ATP in 250 µl of reaction solutions containing 100 mM Bicine-KOH buffer (pH 8.5), 50 mM sodium bicarbonate, 2-oxoglutarate, Mg-ATP, 18 µg of isocitrate dehydrogenase (a recombinant enzyme of Hydrogenobacter thermophilus TK-6 origin, isolated from E. coli), 0.2 M NADH and 3.7 µg of the carboxylation promoting protein at 70°C. After 5 minutes of preincubation without NADH and the carboxylation promoting protein, NADH and the carboxylation promoting protein were added to start the reaction, and the decrease in absorbance at 340 nm (during initial 1 minute) was measured. The activity to oxidize 1 µmol of NADH in 1 minute is defined as 1 unit.

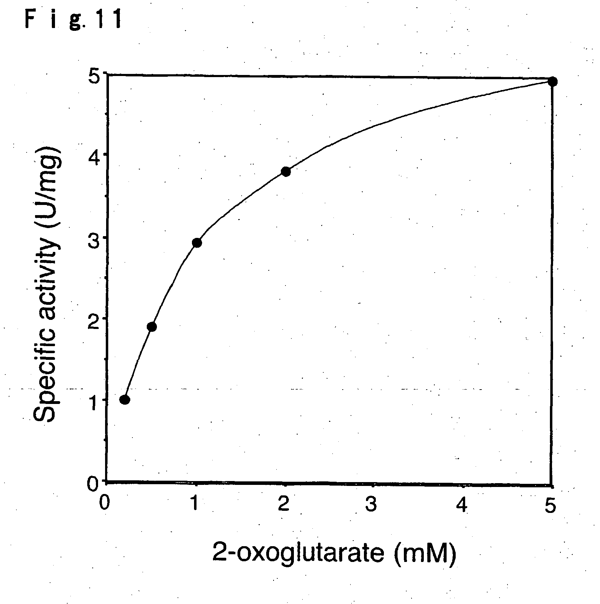

The activity was determined at a constant Mg-ATP concentration of 10 mM at various 2-oxoglutarate concentrations from 0.2 mM to 5 mM, and as a result, the Michaelis-Menten curve shown in Fig. 11 was obtained. From the curve, kinetic parameters were calculated at Km 1.03 mM and Vmax 5.92 U/mg.

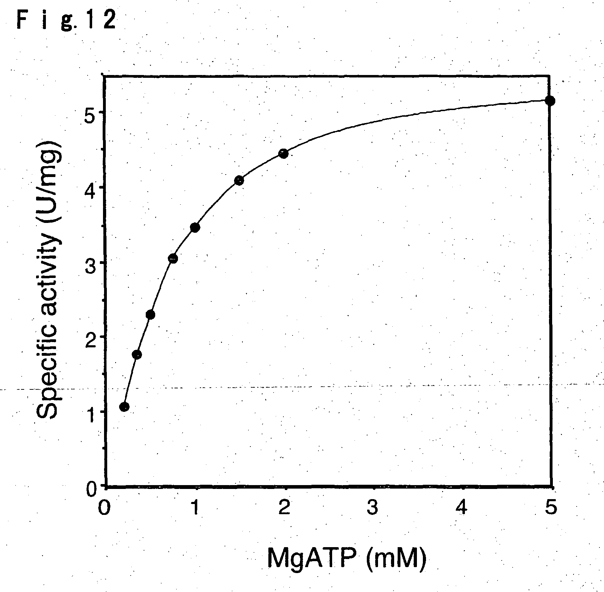

The activity was determined at a constant 2-oxoglutarate concentration of 10 mM at various Mg-ATP concentrations from 0.2 mM to 5 mM, and as a result, the Michaelis-Menten curve shown in Fig. 12 was obtained. From the curve, kinetic parameters were calculated at Km 0.789 mM and Vmax 6.13 U/mg.

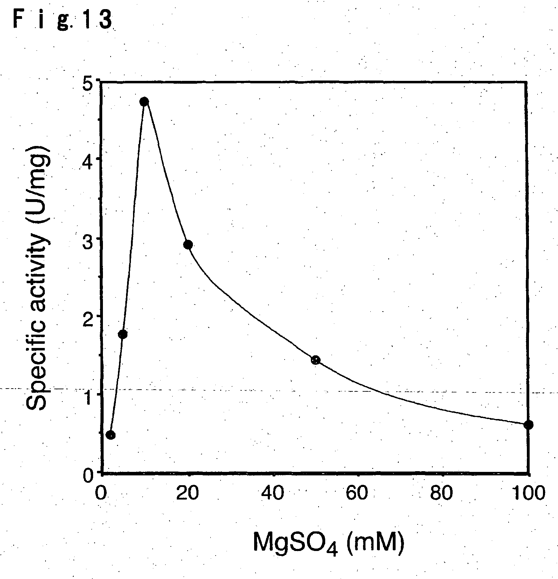

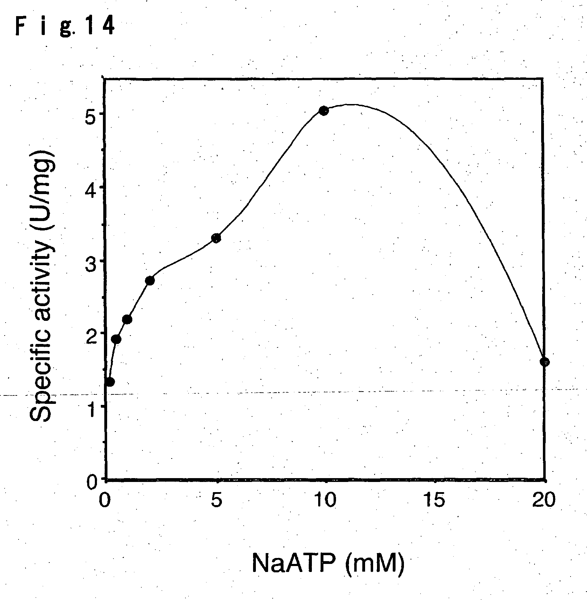

Mg ions and ATP were added separately as magnesium sulfate and NaATP, not simultaneously as Mg-ATP. The activity was determined at a constant NaATP concentration of 10 mM at various magnesium sulfate concentrations from 2 mM to 100 mM, and as a result, the curve shown in Fig. 13 which is not a Michaelis-Menten curve was obtained. The optimum Mg ion concentration was 10 mM. High activity was observed only within a narrow Mg ion concentration range, and the activity was remarkably low below and over 10 mM. The activity was determined at a constant magnesium sulfate concentration of 10 mM at various NaATP concentrations from 0.2 mM to 20 mM, and as a result, the curve shown in Fig. 14 which is not a Michaelis-Menten curve was obtained. The optimum Mg ion concentration was 10 mM, and the activity was low below and over 10 mM.

For the recombinant carboxylation promoting protein isolated from E. coli and the carboxylation promoting protein isolated from Hydrogenobacter thermophilus TK-6, the same kinetic parameters were obtained, and the same Mg- and ATP-dependency profiles were observed.

### INDUSTRIAL APPLICABILITY

The global warming due to increasing carbon dioxide emissions has been causing serious global environmental problems. Under these circumstances, an attempt to utilize carbon dioxide as a starting material in substance production is important in view of resources and energy problems and environmental problems.

Isocitrate dehydrogenase is a decarboxylation enzyme widely distributed in the living world. Though it is reported that the enzyme of certain origin has somewhat of reversibility, the reverse reaction (carboxylation) is disadvantageous in terms of energy and hardly proceeds. The present invention is based on the discovery of a protein which proceeds the reaction catalyzed by the enzyme. Promotion of enzymatic reaction by isocitrate dehydrogenase toward carboxylation according to the present invention is applicable in techniques for biomass production using carbon dioxide and would help solve carbon dioxide problems.

The entire disclosure of Japanese Patent Application No. 2005-042671 filed on February 18, 2005 including specification, claims, drawings and summary is incorporated herein by reference in its entirety.

## Claims

1. A carboxylation promoting protein which promotes reductive carboxylation of 2-oxoglutarate to isocitrate by isocitrate dehydrogenase and contains two subunits having a molecular weight of about 72 kDa and a molecular weight of about 49 kDa.

2. An about 72-kDa carboxylation promoting protein subunit having any of the following sequences (1) to (4):
(1) a polypeptide having an amino acid sequence shown as SEQ ID NO:1;
(2) a polypeptide containing the polypeptide as identified above in (1);
(3) a polypeptide having an amino acid sequence shown as SEQ ID NO:1 in which at least one amino acid is substituted, deleted, inserted and/or added.
(4) a polypeptide having an amino acid sequence which is at least 70% homologous to SEQ ID NO:1.

3. A DNA having any one of the following sequences (1) to (3) :
(1) a polynucleotide encoding the polypeptide as identified in Claim 2 or its complementary strand:
(2) a nucleic acid sequence shown as or complementary to SEQ ID NO:2: and
(3) a DNA hybridizable to the DNA as identified above in (1) or (2) under stringent conditions.

4. An about 49-kDa carboxylation promoting protein subunit having a carbon dioxide fixing ability, which has any one of the following sequences (1) to (4):
(1) a polypeptide having an amino acid sequence shown as SEQ ID NO:3;
(2) a polypeptide containing the polypeptide as identified in (1);
(3) a polypeptide having an amino acid sequence shown as SEQ ID NO:3 in which at least one amino acid is substituted, deleted, inserted and/or added; and
(4) a polypeptide having an amino acid sequence at least 70% homologous to SEQ ID NO:3.

5. A DNA having a sequence selected from the following (1) to (3):
(1) a polynucleotide encoding the polypeptide as identified above in 4 or its complementary strand;
(2) a DNA having a nucleic acid sequence shown as SEQ ID NO:4 or its complementary strand; and
(3) a DNA hybridizable to the DNA as identified above in (1) or (2) under stringent conditions.

6. A vector carrying the DNA as identified in Claim 3, the DNA as identified in Claim 5, or both the DNA as identified in Claim 3 and the DNA as identified in Claim 5.

7. A transformant carrying the DNA as identified in Claim 3, the DNA as identified in Claim 5, both the DNA as identified in Claim 3 and the DNA as identified in Claim 5, or the vector as identified above in Claim 6.

8. A method for producing a carboxylation promoting protein, which comprises culturing the transformant as identified in Claim 7 and recovering an expressed protein.

9. A carboxylation promoting protein which comprises the carboxylation promoting protein subunit as identified in Claim 2 and the carboxylation promoting protein subunit as identified in Claim 4.

10. A carbon dioxide fixing method which comprises promoting reductive carboxylation by isocitrate dehydrogenase using the carboxylation promoting protein as identified above in Claim 1 or Claim 9.
